# EUROPÄISCHE PATENTANMELDUNG

(11) **EP 2 017 350 A1**
(43) Veröffentlichungstag der Anmeldung: **21.01.2009**
(21) Anmeldenummer: 07014218.7
(22) Anmeldetag: 19.07.2007
(51) Int. Cl.: C12Q 1/00

(54) **Elektrochemischer Sensor mit kovalent gebundenem Enzym**

(71) Anmelder: F. Hoffmann-La Roche AG, 4070 Basel (CH); Roche Diagnostics GmbH, 68305 Mannheim (DE)
(72) Erfinder: Die Erfindernennung liegt noch nicht vor
(74) Vertreter: Weiss, Wolfgang

(57) **Zusammenfassung**

Die vorliegende Anmeldung betrifft einen elektrochemischen Sensor, ein Verfahren zu dessen Herstellung sowie ein Verfahren zur Bestimmung eines Analyten in einem fluiden Medium unter Verwendung des elektrochemischen Sensors.

## Beschreibung

Die vorliegende Anmeldung betrifft einen elektrochemischen Sensor, ein Verfahren zu dessen Herstellung sowie ein Verfahren zur Bestimmung eines Analyten in einem fluiden Medium unter Verwendung des elektrochemischen Sensors.

Messsysteme zur biochemischen Analytik stellen wichtige Bestandteile klinisch relevanter Analyseverfahren dar. Hierbei steht die Messung von Analyten im Vordergrund, welche mit Hilfe von Enzymen direkt oder indirekt bestimmt werden. Als zur Messung von Analyten besonders geeignet haben sich Biosensoren, d. h. mit biologischen Komponenten ausgestattete Messsysteme, erwiesen, welche kontinuierlich oder diskontinuierlich eine wiederholte Messung des Analyten gestatten und sowohl *ex vivo* als auch *in vivo* angewendet werden können. Typischerweise werden *ex vivo-*Biosensoren in Durchflusszellen eingesetzt, während *in vivo*-Biosensoren vorzugsweise in subkutanes Fettgewebe implantiert werden. Hierbei unterscheidet man transkutane Implantate, welche lediglich für kurze Zeit in das Gewebe eingebracht werden und in direktem Kontakt mit einer auf der Haut befindlichen Messeinrichtung stehen, und Vollimplantate, welche zusammen mit einer Messeinrichtung operativ in das Gewebe eingesetzt werden.

Elektrochemische Biosensoren gestatten die Messung eines Analyten mittels zweier oder mehrerer Elektroden, wobei mindestens eine der Elektroden die Arbeitselektrode darstellt, an welcher der zu bestimmende Analyt umgesetzt wird. Elektrochemische Biosensoren, welche ein Enzym als biologische Komponente umfassen, enthalten das Enzym in oder auf der Arbeitselektrode, wobei z. B. der Analyt dem Enzym als Substrat dienen und von diesem physikochemisch verändert (z. B. oxidiert) werden kann. Ein Redoxmediator überträgt die bei der Umsetzung des Analyten freigesetzten Elektronen auf die leitfähigen Bestandteile der Arbeitselektrode, wobei das durch den Elektronenfluss erzeugte elektrische Messsignal mit der Konzentration des gemessenen Analyten korreliert.

Als Redoxmediatoren kommen sowohl natürlich vorkommende als auch synthetische Redoxpaare in Betracht. Für *in vivo*-Anwendungen sind synthetische Redoxmediatoren, wie sie beispielsweise von Feldman et al. [Diabetes Technology & Therapeutics 5 (2003), 769-779] beschrieben werden, allerdings wenig geeignet. Dies liegt darin begründet, dass ein synthetischer Redoxmediator theoretisch stets eine Immunantwort des Körpers bei Einbringung des Biosensors in den Körper hervorrufen kann. Zumindest jedoch muss die Toxizität dieser Substanzen beachtet und gegebenenfalls überprüft werden, da Redoxmediatoren prinzipiell frei durch die Elektrodenstruktur diffundieren können müssen, womit sie auch aus der Elektrode austreten bzw. in den sie umgebenden Organismus übertreten können. Bei *ex vivo*-Anwendungen ist dieser Punkt nicht relevant, sofern sichergestellt ist, dass es nicht über einen potentiellen Rückfluss des Analyten zum Eintritt in den Körper kommt.

Folglich eignen sich für *in vivo*-Anwendungen insbesondere elektrochemische Sensoren, welche sich natürlich vorkommender Redoxmediatoren bedienen. Das Redoxpaar Sauerstoff/Wasserstoffperoxid erweist sich in diesem Zusammenhang als besonders vorteilhaft, da bereits der Initialteil (Sauerstoff) stets vorhanden ist. Das im Rahmen der enzymatischen Umsetzung eines Analyten mittels einer Oxidase in Gegenwart von Sauerstoff erzeugte Wasserstoffperoxid wird an der Arbeitselektrode des elektrochemischen Biosensors reoxidiert, wobei durch Abgabe von Elektronen ein elektrisches Signal erzeugt und der Redoxmediator in seine oxidierte Form zurückversetzt wird. Die Kinetik dieser enzymatischen Reaktion folgt einem sogenannten Ping-Pong-Mechanismus [Leskovac et al., The International Journal of Biochemistry and Cell Biology 37 (2005), 731-750].

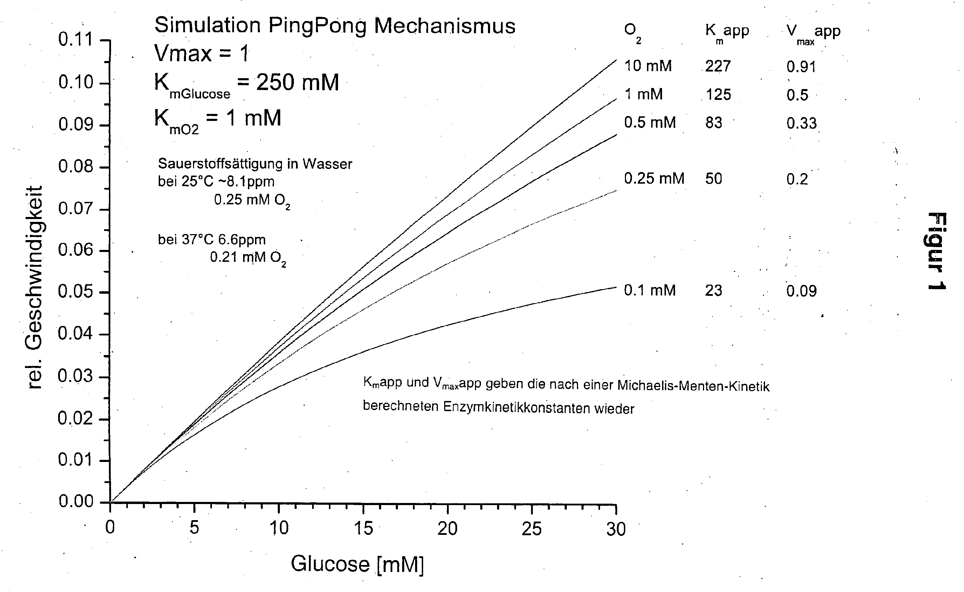

Ein nicht unerhebliches Problem bei der Messung von Analyten mit Hilfe von Enzymen, welche Sauerstoff als Co-Substrat benötigen, ist allerdings, dass es in Geweben zu temporären Verringerungen der Sauerstoffkonzentration gegenüber der Ausgangslage kommen kann, wodurch die Funktion herkömmlicher *in vivo*-Biosensoren beeinflusst werden kann. Figur 1 zeigt die Kinetik der enzymatischen Oxidation von Glucose zu Glucono-δ-lacton mittels Glucose-Oxidase bei verschiedenen Sauerstoffkonzentrationen. Wie der Grafik zu entnehmen ist, verringert sich mit steigender Glucosekonzentration allgemein die vom Enzym bei einer vorgegebenen Sauerstoffkonzentration umgesetzte Menge an Analyt, womit die Kurve im physiologisch relevanten Bereich trotz der hohen Bindungskonstante von Glucose-Oxidase für Glucose (ca. 250 mM) im nicht-linearen Bereich liegt.

Ferner zeigt Figur 1, dass bei höheren Konzentrationen des Analyten erst bei einer Sauerstoffkonzentration von etwa 1 mM eine annähernd lineare Kurve erhalten wird. Die *in vivo*-Konzentration an gelöstem Sauerstoff in wässrigen Systemen und insbesondere in der interstitiellen Flüssigkeit des subkutanen Fettgewebes liegt indessen deutlich niedriger. Während Wasser bei 37°C etwa eine Sauerstoffkonzentration von 0.21 mM aufweist, liegt die für subkutanes Fettgewebe zu erwartende Sauerstoffkonzentration bei lediglich 0.1 mM oder gar niedriger, womit die Kurven bei physiologischen Glucosekonzentrationen jeweils gekrümmt sind. Diese Abweichung vom linearen Verlauf führt bei *in vivo*-Biosensoren zu einer unerwünschten transienten Funktionscharakteristik.

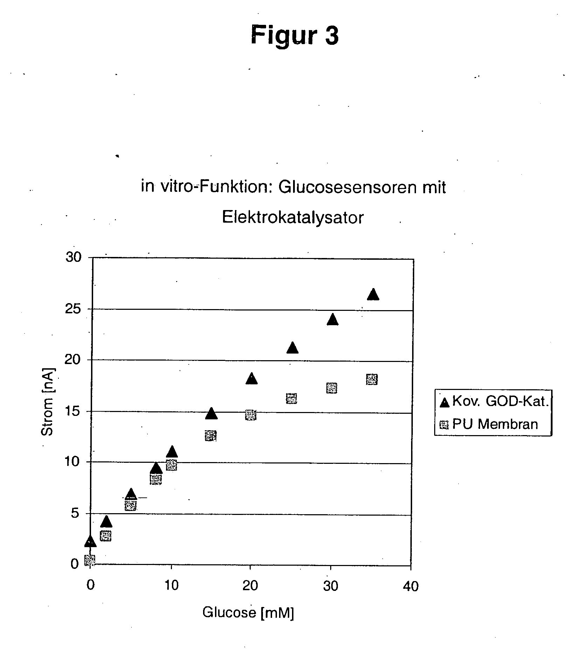

In einer Vielzahl enzymatischer Biosensoren, welche Sauerstoff als Co-Substrat benötigen, stellt somit die begrenzte Verfügbarkeit von Sauerstoff im Gewebe den limitierenden Faktor für die Linearität der Funktionskurve des elektrochemischen Sensors dar. Die Linearität der Funktionskurve lässt sich prinzipiell durch Verwendung von Arbeitselektroden mit einer Deckmembran verbessern, welche die Diffusion des Analyten stärker hemmt als die Diffusion des Co-Substrats. Figur 3 zeigt u.a. die Funktionskurve eines enzymatischen Biosensors mit einer aus Polyurethan bestehenden Deckmembran, welche eine Diffusion von Sauerstoff stärker begünstigt als jene von Glucose (als Quadrate gekennzeichnete Messwerte). Hierbei zeigt sich, dass das Messsignal des Sensors durch Verwendung einer geeigneten Deckmembran bis zu einer Glucosekonzentration von etwa 10 mM annähernd linear gehalten werden kann. Bei höheren Konzentrationen ist die Kurve in zunehmendem Maße gekrümmt.

Allerdings ist auch der Einsatz von Deckmembranen in elektrochemischen Sensoren mit gewissen Problemen verbunden. So müssen elektrochemische Sensoren, welche der Bestimmung verschiedener Analyten dienen, in der Regel auch verschiedene Deckmembranen enthalten, um eine unterschiedliche Diffusion von Substrat und Co-Substrat bereitzustellen. Gleichzeitig muss bei *in vivo*-Anwendungen eine hohe Biokompatibilität der Deckmembranen gewährleistet sein, was erhebliche technische Anforderungen mit sich bringt und letztlich zu erhöhten Produktionskosten führt.

Um die Polarisationsspannung der Arbeitselektrode eines elektrochemischen Biosensors gegenüber einer Referenzelektrode zu senken und damit den Einfluss von interferierenden Substanzen auf das Messsignal der Arbeitselektrode zu senken, bedienen sich einige elektrochemische Biosensoren zusätzlich eines Elektrokatalysators, welcher die Übertragung von Elektronen vom Redoxmediator auf die leitfähigen Bestandteile der Arbeitselektrode begünstigt. Ein Beispiel für einen solchen Elektrokatalysator ist Cobalt-Phthalocyanin, welches die Oxidation von Wasserstoffperoxid zu Sauerstoff katalysiert [Crouch et al., Biosensors and Bioelectronics 21 (2005), 712-718]. Hierbei wird das Cobalt (II)-Kation des Cobalt-Phthalocyanin-Komplexes zunächst von Wasserstoffperoxid zu Cobalt (I) reduziert, bevor dieses unter Abgabe eines Elektrons an der Anode in den urprünglichen zweiwertigen Zustand zurückversetzt wird.

Ein weiteres aus der Literatur bekanntes Beispiel für einen Elektrokatalysator ist Mangandioxid in Form von Braunstein [Cui et al., Nanomedicine: Nanotechnology, Biology and Medicine 1 (2005), 130-135; Luo et al., Biosensors and Bioelectronics 19 (2004), 1295-1300]. Obgleich der Mechanismus der katalytischen Oxidation von Wasserstoffperoxid an Braunstein nicht im Detail verstanden wird, wird das Potential einer Arbeitselektrode mit Braunstein als Elektrokatalysator gegenüber einer Arbeitselektrode ohne Braunstein um mehrere 100 mV erniedrigt. Infolgedessen reduziert sich der Einfluss von interferierenden Substanzen, wie beispielsweise Ascorbat oder Harnstoff, auf das Messsignal erheblich.

Ein weiterer Grund für die Verwendung von Elektrokatalysatoren liegt in der Schädigung von Enzymen durch überschüssiges Wasserstoffperoxid. Wird diese Substanz nicht ausreichend schnell an der Arbeitselektrode zersetzt, kann es zu einer Denaturierung des Enzyms kommen. Um diesem Problem zu begegnen, wurde in der Literatur vorgeschlagen, Enzyme mit einer Resistenz gegenüber Wasserstoffperoxid, beispielsweise durch Mutation, zu synthetisieren [U.S. 2004/0137547 A1]. Allerdings bereitet es erhebliche Schwierigkeiten, derartige Veränderungen an einem Enzym vorzunehmen, ohne dabei andere Eigenschaften des Enzyms negativ zu beeinflussen, wie beispielsweise dessen enzymatische Spezifität. Daher erscheint der Einsatz von Elektrokatalysatoren im Rahmen von Umsetzungen, bei welchen Wasserstoffperoxid erzeugt wird, obigem Verfahren deutlich überlegen, da Elektrokatalysatoren die Effizienz der Oxidation von Wasserstoffperoxid erheblich steigern und auf diese Weise Überschüsse von Peroxid in der Elektrodenmatrix oder in deren Umgebung verhindern.

Ein zusätzliches Problem, welches mit der Bildung von Wasserstoffperoxid im Rahmen der enzymatischen Bestimmung eines Analyten verbunden ist, ist jenes, dass Wasserstoffperoxid als Inhibitor des Analyten oder des Co-Substrats Sauerstoff fungieren kann. Diese kompetitive Hemmung zeigt eine Abhängigkeit von der Konzentration des Wasserstoffperoxids und führt zu einer Einschränkung des Umsatzes an Analyt. Die Verwendung eines Elektrokatalysators, welcher die Reoxidation von Wasserstoffperoxid zu Sauerstoff fördert, wirkt sich folglich auch in Bezug auf den Umsatz des Analyten positiv aus.

Im Rahmen der Konzipierung elektrochemischer Biosensoren sind verschiedene Faktoren zu berücksichtigen. So müssen die Biosensoren eine ausreichende Menge an Enzym in der Arbeitselektrode aufweisen, um eine Enzymlimitierung der Messung zu verhindern [Abel et al., Journal of Molecular Catalysis B: Enzymatic 7 (1999), 93-100]. Ferner sollten die Enzymmoleküle über die komplette Messdauer des Biosensors in der Struktur der Arbeitselektrode lokalisiert sein, d. h. es sollte nicht zu Ablösungen und Verlagerungen des Enzyms in Bereichen der Elektrode kommen, die vom Messmedium erreicht werden [Doretti et al., Biosensors and Bioelectronics 11 (1996), 363-373]. Schließlich sollte das Enzym in der Arbeitselektrode des Biosensors auch stabil sein. Faktoren, welche die thermische Deaktivierung von Enzymen in elektrochemischen Biosensoren bewirken, sind, gemeinsam mit Methoden zur Stabilisierung, vielfach untersucht worden [Sarath Babu et al., Biosensors and Bioelectronics 19 (2004), 1337-1341]. Der Enzymabbau nach Herstellung eines Biosensors führt schlussendlich zu einer eingeschränkten Lagerfähigkeit des Sensors.

Um obigen Faktoren Rechnung zu tragen, wurde eine Stabilisierung des Enzyms durch Immobilisierung in der Elektrodenmatrix der Arbeitselektrode angestrebt, was zu einer intensiven Suche nach geeigneten Immobilisierungsverfahren für Enzyme in elektrochemischen Biosensoren geführt hat. In der Praxis kommen sowohl eine adsorptive als auch eine chemische Immobilisierung zur Anwendung. Die adsorptive Immobilisierung ist jedoch in verschiedener Hinsicht nachteilig. Zum einen erfordert sie eine Abdeckung der Arbeitselektrode mittels einer für das Enzym undurchlässigen Membran, was den Aufwand bei der Herstellung des Biosensors erhöht und vielfältige Anforderungen an die Membran stellt. Andererseits kann die vorstehend genannte Verlagerung von Enzymmolekülen innerhalb der Elektrode bei adsorptiver Immobilisierung nicht unterbunden werden, was zu einer Veränderung der Sensorfunktion führt.

U.S.5,368,707 offenbart Biosensoren, welche Arbeitselektroden mit adsorptiv gebundenem Enzym umfassen und zur Bestimmung mikromolarer Mengen an Bleiionen in Flüssigkeiten geeignet sind. Zur Herstellung der Biosensoren wird die aus einem leitfähigen Material bestehende Oberfläche der Arbeitselektrode mit kolloidalem Gold beschichtet, an dessen Partikel das entsprechende Enzym, welches seinerseits kovalent an einen Redoxmediator gebunden sein kann, adsorbiert ist.

Ein weiterer, insbesondere für *in vivo*-Anwendungen nicht zu unterschätzender Nachteil von Elektroden, welche eine Deckmembran zur Unterstützung der adsorptiven Immobilisierung von Enzymen vorsehen, besteht in der erforderlichen nicht-invasiven Prüfung der Integrität der Deckmembran. Da selbst kleinste Fehlstellen in der Membran genügen, um ein Ausbluten des Enzyms aus der Elektrode in die Umgebung zu bewirken, ist insbesondere bei *in vivo*-Biosensoren ein enormer Prüfaufwand erforderlich. In Anbetracht der Nachteile einer adsorptiven Immobilisierung besteht somit ein konkretes Bedürfnis, Enzyme in elektrochemischen Biosensoren über kovalente Bindungen an oder in der Elektrodenmatrix zu fixieren.

JP 10-68651 beschreibt Sensoren zur Detektion von Analyten wie Glucose, welche Elektroden mit kovalent gebundenem Enzym umfassen. Zu diesem Zweck wird die mit SnO₂ als leitfähigem Material beschichtete Oberfläche der Elektrode mit einer starken Säure aktiviert, mit einem Kopplungsreagenz funktionalisiert und abschließend mit dem Enzym in Kontakt gebracht.

EP 0 247 850 A1 offenbart Biosensoren zur amperometrischen Detektion eines Analyten. Diese Sensoren enthalten Elektroden mit immobilisierten Enzymen, welche an der Oberfläche eines elektrisch leitenden Trägers immobilisiert oder adsorbiert sind, wobei der Träger aus einer platinierten porösen Schicht aus harzgebundenen Kohlenstoff- oder Graphitkörnern besteht oder eine solche Schicht enthält. Zu diesem Zweck werden zunächst Elektroden aus platiniertem Graphit und einem polymeren Bindemittel bereitgestellt, und diese anschließend mit dem Enzym in Kontakt gebracht. Die Immobilisierung des Enzyms erfolgt hierbei entweder durch Adsorption an die Elektrodenoberfläche oder durch Kopplung an das polymere Bindemittel unter Verwendung geeigneter Reagenzien.

Amperometrische Biosensoren mit Elektroden, umfassend ein auf bzw. in einem elektrisch leitenden, porösen Elektrodenmaterial immobiliertes oder adsorbiertes Enzym, sind auch in EP 0 603 154 A2 beschrieben. Zur Herstellung der Enzymelektroden wird ein als Katalysator fungierendes Oxid oder Oxidhydrat eines Übergangsmetalls der vierten Periode, wie beispielsweise Mangandioxid, mit Graphit und einem nicht leitenden polymeren Bindemittel zu einer Paste verarbeitet, wobei das nach Trocknen der Paste erhaltene poröse Elektrodenmaterial in einem zweiten Schritt mit dem Enzym in Kontakt gebracht wird. Eine Immobilisierung des Enzyms auf bzw. in dem porösen Elektrodenmaterial kann durch Vernetzung mittels Glutardialdehyd erfolgen.

Ein wesentlicher Nachteil der in JP 10-68651, EP 0 247 850 A1 und EP 0 603 154 A2 beschriebenen elektrochemischen Biosensoren besteht darin, dass die Immobilisierung des Enzyms erst an der ohne Enzym vorgefertigten Elektrode erfolgt. Dies bringt das Problem mit sich, dass die Ankopplung des Enzyms an die Elektrodenbestandteile nicht kontrolliert werden kann. So bindet das Enzym bei Verwendung von Glutardialdehyd als Vernetzungsreagenz nicht nur unkontrolliert an beliebige reaktive Bestandteile des Elektrodenmaterials, sondern vernetzt auch untereinander. Darüber hinaus verursacht diese Vorgehensweise eine Kontamination der Elektrode mit den eingesetzten Reagenzien, womit die Elektrode insbesondere vor Verwendung in einem *in vivo*-Biosensor nochmals gründlich gereinigt werden muss, was den Produktionsaufwand und somit die Kosten erhöht.

U.S. 4,938,860 offenbart eine für elektrochemische Sensoren geeignete Elektrode, umfassend eine mit Platin beschichtete, als Film ausgebildete Anode und eine Enzymschicht, welche an die Anode gebunden ist. Die Bindung der Enymschicht an die platinierte Anode erfolgt bevorzugt unter Verwendung eines Aminosilans und eines geeigneten Vernetzungsmittels, wie beispielsweise Glutardialdehyd. Ein Nachteil der in U.S. 4,938,860 beschriebenen Elektrode besteht jedoch darin, dass aufgrund der Ausbildung der Anode als Film lediglich eine geringe Oberfläche für die enzymatische Umsetzung des Analyten bereitgestellt und mit Platin als Katalysator ein vergleichsweise teures Material verwendet wird.

Die der Erfindung zugrunde liegende Aufgabe bestand deshalb darin, einen enzymatischen elektrochemischen Sensor zur Bestimmung eines Analyten bereitzustellen, bei welchem die Nachteile des Standes der Technik zumindest teilweise beseitigt sind. Insbesondere sollte der Sensor eine gezielte und dauerhafte Immobilisierung des Enzyms gewährleisten, eine hohe Effizienz besitzen und damit eine hohe Signalausbeute erreichen. Weiterhin sollte der Sensor einfach und kostengünstig hergestellt werden können.

Diese Aufgabe wurde erfindungsgemäß gelöst mittels eines elektrochemischen Biosensors zur Bestimmung eines Analyten in einem fluiden Medium, umfassend mindestens eine Arbeitselektrode und mindestens eine Referenzelektrode, wobei mindestens die Arbeitselektrode Partikel eines Elektrokatalysators in einer Elektrodenmatrix umfasst, wobei ein zur Bestimmung des Analyten geeignetes Enzym kovalent an die Partikel des Elektrokatalysators gebunden ist.

Als Elektrokatalysator, welcher in der Elektrodenmatrix zumindest der Arbeitselektrode vorhanden ist, dient bevorzugt ein Metalloxid. Das Metalloxid kann ein beliebiges Metalloxid sein, welches die Fähigkeit besitzt, die Umsetzung eines für die Bestimmung des Analyten verwendeten Redoxmediators zu katalysieren. In einer stärker bevorzugten Ausführungsform handelt es sich bei dem verwendeten Elektrokatalysator um ein Metalloxid ausgewählt aus der Gruppe bestehend aus MnO₂, FeOOH, Fe₃O₄, Fe₂O₃, Cr₂O₃ und V₂O₅, wobei MnO₂ als besonders bevorzugt gilt. Wünschenswerterweise besitzt der Elektrokatalysator darüber hinaus eine hohe Affinität zu Sauerstoff.

Der Elektrokatalysator wird erfindungsgemäß in partikulärer Form bereitgestellt, wobei die Partikelgröße entsprechend den jeweiligen Anforderungen variiert werden kann. Im Rahmen der vorliegenden Erfindung weisen 90% der Elektrokatalysatorpartikel üblicherweise einen Durchmesser von 0.1 µm bis 20 µm auf, wobei sich ein Durchmesser von 0.5 µm bis 5 µm als bevorzugt erwiesen hat. In jedem Fall sollte die Partikelgröße des Elektrokatalysators stets kleiner sein als die Schichtdicke der Arbeitselektrode, welche im Bereich von 1 µm bis 50 µm, bevorzugt im Bereich von 5 µm bis 20 µm, liegt.

Die Steuerbarkeit der effektiven Oberfläche des Elektrokatalysators mittels der Partikelgröße ist insbesondere für dessen Funktionalisierung mit Enzym von entscheidender Bedeutung. So kann durch eine höhere effektive Oberfläche des Elektrokatalysators auch dessen Beladung mit Enzym erhöht und damit eine höhere Enzymaktivität, angegeben in Units pro Milligramm Elektrokatalysator, erzielt werden, welche im Allgemeinen durch die Menge an Elektrokatalysator in der Arbeitselektrode sowie deren Porosität und Fläche bestimmt wird. Der Begriff "Unit", wie er im Rahmen der vorliegenden Anmeldung verwendet wird, stellt dabei jene Menge an Enzym dar, welche erforderlich ist, um unter Standardbedingungen 1 µmol Substrat pro Minute umzusetzen. Die zum Zwecke der vorliegenden Erfindung verwendeten enzymbeschichteten Elektrokatalysatorpartikel weisen üblicherweise eine Enzymaktivität von etwa 0.01 U/mg bis etwa 10 U/mg auf, wobei sich eine Enzymaktivität von etwa 0.1 U/mg bis etwa 10 U/mg als besonders vorteilhaft erwiesen hat.

Vorzugsweise liegt beim erfindungsgemäßen elektrochemischen Sensor eine selektive kovalente Bindung des Enzyms an die Partikel des Elektrokatalysators vor, wobei das Enzym besonders bevorzugt keine kovalenten Bindungen zu den übrigen Bestandteilen der Elektrodenmatrix aufweist. Die kovalente Bindung des Enzyms an den Elektrokatalysator besitzt den Vorteil, dass die Diffusionsstrecke des Redoxmediators zu katalytisch aktiven Stellen der Elektrode klein gehalten wird, womit eine hohe Effizienz der Arbeitselektrode und damit eine hohe Signalausbeute des elektrochemischen Sensors erreicht wird.

Weiterhin wird der Redoxmediator nach Regeneration durch den Elektrokatalysator auch adsorptiv an diesen gebunden, wodurch beispielsweise im Falle des Sauerstoff/Wasserstoffperoxid-Systems eine lokal hohe Sauerstoffaktivität im Bereich der Oberfläche des Elektrokatalysators entsteht, die zum umgebenden Messmedium hin abfällt. Die kovalente Bindung des Enzyms an den Elektrokatalysator bewirkt andererseits eine hohe lokale Aktivität des regenerierten Redoxmediators am Enzym, was sich in einer höheren Linearität und Stabilität des erzeugten Messsignals in Bezug auf die Konzentration an Analyt widerspiegelt, wie beispielsweise aus Figur 3 hervorgeht (als Dreiecke gekennzeichnete Messwerte). In diesem Fall führt auch eine vorübergehende Absenkung der Konzentration des Redoxmediators in der Umgebung, z. B. durch eine verschlechterte Durchblutung des Gewebes, nicht zu einer transienten Veränderung des Messsignals.

Schließlich wird durch die kovalente Kopplung des Enzyms an den Elektrokatalysator die Konstanz der Funktion gewährleistet, da eine Ablösung von Enzymmolekülen bei den typischen Messbedingungen (physiologische Elektrolytkonzentration, physiologischer pH, Körpertemperatur) ausgeschlossen werden kann. Somit bleibt der erfindungsgemäße elektrochemische Sensor über einen langen Zeitraum betriebsfähig und operiert praktisch frei von Drift.

Um eine kovalente Bindung des Enzyms an die Partikel des Elektrokatalysators zu bewirken, sieht die vorliegende Erfindung in einer bevorzugten Ausführungsform vor, dass die Elektrokatalysatorpartikel eine funktionalisierte Oberfläche, und insbesondere eine mit Aminogruppen oder/und Carboxylgruppen funktionalisierte Oberfläche, aufweisen, an die das Enzym gebunden ist. Die Funktionalisierung der Oberfläche kann beispielsweise durch Beschichtung der Elektrokatalysatorpartikel mit einem geeigneten Reagenz erfolgen, wobei auf der Oberfläche der Elektrokatalysatorpartikel funktionelle Gruppen ausgebildet werden, über welche eine kovalente Bindung des Enzyms an die Elektrokatalysatorpartikel erfolgen kann.

Beschichtungsreagenzien, welche im Rahmen der vorliegenden Erfindung Anwendung finden, sind Substanzen, die einerseits eine kovalente Bindung mit dem Elektrokatalysator, z. B. mit Hydroxygruppen des Elektrokatalysators, eingehen und andererseits mindestens eine funktionelle Gruppe enthalten, die zur kovalenten Bindung des Enzyms dient. Dies bedeutet, dass die Beschichtungsreagenzien mindestens bifunktionell sind, d. h. mindestens zwei funktionelle Gruppen umfassen. Die zur kovalenten Bindung an den Elektrokatalysator und die zur kovalenten Bindung an das Enzym dienenden funktionellen Gruppen des Beschichtungsreagenzes können gleich oder verschieden sein, sind jedoch bevorzugt verschieden. Bevorzugte Beschichtungsreagenzien sind Silane, welche mindestens eine geeignete funktionelle Gruppe tragen, über die das Enzym kovalent an das Beschichtungsreagenz gebunden wird.

Stärker bevorzugt ist die Oberfläche der Elektrokatalysatorpartikel mit einem Aminosilan funktionalisiert, welches unter Ausbildung von Silizium-Sauerstoff-Bindungen an die Oberfläche der Elektrokatalysatorpartikel bindet und gleichzeitig freie Aminogruppen für eine kovalente Bindung des Enzyms an die Elektrokatalysatorpartikel bereitstellt. Geeignete Aminosilane umfassen beispielsweise 3-Aminopropyltrimethoxysilan und 3-Aminopropyltriethoxysilan, wobei 3-Aminopropyltriethoxysilan besonders bevorzugt ist.

Alternativ kann die Oberfläche der Elektrokatalysatorpartikel mit einem Carboxysilan funktionalisiert sein, welches unter Ausbildung von Silizium-Sauerstoff-Bindungen an die Oberfläche der Elektrokatalysatorpartikel bindet und, gegebenenfalls nach Hydrolyse, freie Carboxylgruppen für eine kovalente Bindung des Enzyms an die Elektrokatalysatorpartikel bereitstellt. Als besonders geeignetes Silan hat sich hierbei 3-(Triethoxysilyl)-propylbernsteinsäureanhydrid erwiesen, welches als Geniosil^{®} GF 20 (Fa. Wacker) im Handel erhältlich ist.

Das Enzym kann direkt oder über Vernetzungsreagenzien kovalent an die funktionalisierte Oberfläche der Elektrokatalysatorpartikel gebunden sein. In einer bevorzugten Ausführungsform ist das Enzym direkt an die funktionalisierte Oberfläche der Elektrokatalysatorpartikel gebunden. Die Kopplung des Enzyms an die funktionalisierte Oberfläche der Elektrokatalysatorpartikel kann in beliebiger Weise erfolgen und eine vorherige Aktivierung funktioneller Gruppen auf der funktionalisierten Oberfläche der Elektrokatalysatorpartikel oder/und des Enzyms umfassen. Eine Aktivierung funktioneller Gruppen kann beispielsweise durch Umsetzung des funktionalisierten Elektrokatalysators oder/und des Enzyms mit einem geeigneten Aktivierungsreagenz bewirkt werden. Bevorzugte Aktivierungsreagenzien umfassen Carbodiimide, wie beispielsweise Dicyclohexylcarbodiimid (DCC), Diisopropylcarbodiimid oder 1-Ethyl-3-(3-dimethylaminopropyl)carbodiimid (EDC), sowie Kombinationen von Carbodiimiden und Succinimiden. Ein für die Zwecke der vorliegenden Erfindung besonders geeignetes Aktivierungsreagenz umfasst eine Kombination von 1-Ethyl-3-(3-dimethylaminopropyl)carbodiimid (EDC) und N-Hydroxysuccinimid.

In einer anderen bevorzugten Ausführungsform ist das Enzym über ein Vernetzungsreagenz an die funktionalisierte Oberfläche der Elektrokatalysatorpartikel gebunden, wobei das Vernetzungsreagenz reaktive Gruppen aufweist, die kovalent sowohl mit den funktionellen Gruppen auf der funktionalisierten Oberfläche der Elektrokatalysatorpartikel als auch mit den funktionellen Gruppen des Enzyms reagieren können. Besonders bevorzugt sind reaktive Gruppen vorhanden, die eine Vernetzung zwischen dem Enzym und den funktionellen Gruppen auf der Oberfläche der Elektrokatalysatorpartikel ermöglichen. Als Vernetzungsreagenzien kommen beliebige Reagenzien in Frage, welche die vorstehend beschriebene Funktion erfüllen können, wie beispielsweise polyfunktionelle Aldehyde, insbesondere Dialdehyde wie Glutardialdehyd, Benzochinone, Bromcyan, Hydrazine, Succinimide, 2,4,6-Trichlor-1,3,5-triazin, oder Kombinationen hiervon. Bevorzugt wird als Vernetzungsreagenz ein Succinimid, stärker bevorzugt ein Disuccinimid, und am stärksten bevorzugt Disuccinimidylsuberat (DSS) eingesetzt.

Die Elektrodenmatrix der Arbeitselektrode kann durch Vermischen der kovalent mit Enzym beschichteten Elektrokatalysatorpartikel mit weiteren Komponenten der Elektrodenmatrix, z. B. einem leitfähigen Elektrodenmaterial, und anschließendes Trocknen des erhaltenen Gemisches erzeugt werden, wobei die Elektrodenmatrix den Elektrokatalysator üblicherweise in einer Menge von etwa 1 Gew.% bis etwa 50 Gew.%, bevorzugt in einer Menge von etwa 5 Gew.% bis etwa 20 Gew.%, enthält.

In einer weiteren bevorzugten Ausführungsform ist die Elektrodenmatrix porös ausgebildet. Die Porösität der Elektrodenmatrix kann u. a. über die Partikelgröße des Elektrokatalysators und anderer Komponenten gesteuert werden, wobei eine hohe Porösität mit einer größeren effektiven Oberfläche der Elektrode und damit einer größeren Kontaktfläche zum Messmedium verbunden ist. Das leitfähige Elektrodenmaterial, welches zur Herstellung der Elektrodenmatrix beispielsweise in Form einer Paste bereitgestellt werden kann, umfasst vorzugsweise leitfähige Feststoffpartikel wie Graphit oder/und Fullerene in Kombination mit einem nicht-leitenden Bindemittel, insbesondere einem nicht-leitenden polymeren Bindemittel, wie beispielsweise einem perfluorierten Polymer wie Nafion.

Das auf den Elektrokatalysatorpartikeln immobilierte Enzym ist vorzugsweise eine Oxidase, und insbesondere Alkohol-Oxidase (1.1.3.13), Arylalkohol-Oxidase (EC 1.1.3.7), Catechol-Oxidase (EC 1.1.3.14), Cholesterol-Oxidase (EC 1.1.3.6), Cholin-Oxidase (1.1.3.17), Galactose-Oxidase (EC 1.1.3.9), Glucose-Oxidase (EC 1.1.3.4), Glycerin-3-phosphat-Oxidase (EC 1.1.3.21) Hexose-Oxidase (EC 1.1.3.5), Malat-Oxidase (EC 1.1.3.3), Pyranose-Oxidase (EC 1.1.3.10), Pyridoxin-4-Oxidase (EC 1.1.3.12) oder Thiamin-Oxidase (EC 1.1.3.23). Besonders bevorzugt handelt es sich bei dem Enzym um Glucose-Oxidase.

Die Referenzelektrode des erfindungsgemäßen elektrochemischen Sensors dient zur Einstellung des Polarisationspotentials der Arbeitselektrode und kann aus einem beliebigen Material bestehen, welches für die Zwecke der vorliegenden Erfindung geeignet ist. Bevorzugt wird als Referenzelektrode eine Silber/Silberchlorid-Elektrode eingesetzt.

Ferner kann der elektrochemische Sensor der vorliegenden Erfindung neben der mindestens einen Arbeitselektrode und der mindestens einen Referenzelektrode mindestens eine Gegenelektrode umfassen, welche bevorzugt als Edelmetallelektrode, und insbesondere als Goldelektrode, ausgebildet ist. Vorzugsweise ist eine als Edelmetallelektrode ausgebildete Gegenelektrode mit einem geeigneten leitfähigen Material, wie beispielsweise einer Paste mit leitfähigen Feststoffpartikein, insbesondere Carbonpaste, beschichtet.

Erfindungsgemäß enthält der elektrochemische Sensor bevorzugt zwei Abschnitte. Der erste Abschnitt, welcher mit dem den Analyten enthaltenden fluiden Medium in Kontakt gebracht werden kann, umfasst die Elektroden, d. h. Arbeitselektrode, Referenzelektrode und gegebenenfalls Gegenelektrode. Dieser Abschnitt ist vorzugsweise mit einem biokompatiblen Überzug versehen. Der biokompatible Überzug erlaubt das Eindringen des Analyten in die Elektrodenmatrix, soll jedoch den Austritt von Elektrodenkomponenten in das umgebende Medium verhindern. In Anbetracht dessen, dass durch kovalente Bindung des Enzyms an den Elektrokatalysator keine Ausblutung des Enzyms aus der Arbeitselektrode bzw. dem elektrochemischen Sensor erfolgt, ist ein biokompatibler Überzug für viele Anwendungen nicht unbedingt erforderlich. So ist ein Einsatz des erfindungsgemäßen elektrochemischen Sensors insbesondere in *in vivo-*Biosensoren auch möglich, wenn der biokompatible Überzug nicht enzymsperrend wirkt. Vielmehr kann in diesem Zusammenhang ein biokompatibler Überzug gewählt werden, welcher eine optimale Interaktion mit dem umgebenden Gewebe oder/und Blut bzw. Serum bietet.

Die Erzeugung biokompatibler Überzüge kann auf unterschiedliche Weise erfolgen. Ein bevorzugtes Verfahren besteht in der Verwendung vorgefertigter Membranen, welche auf den elektrochemischen Sensor aufgebracht werden. Eine Fixierung der Membran auf dem Sensor kann hierbei mittels beliebiger Techniken erfolgen, wobei Verklebung oder Laserverschweißung als bevorzugt anzusehen sind. Als vorteilhaft haben sich in diesem Zusammenhang vorgefertigte Dialysemembranen erwiesen, wobei Dialysemembranen aus Polyethersulfon, wie sie beispielsweise in EP 1 710 011 A1 offenbart und unter der Handelsbezeichnung Ultrason^{®} 6020 (Fa. BASF) kommerziell erhältlich sind, besonders geeignet sind.

Alternativ besteht die Möglichkeit, den biokompatiblen Überzug *in situ* zu erzeugen, indem eine Lösung eines geeigneten Polymers auf den elektrochemischen Sensor aufgebracht und anschließend getrocknet wird. Das Aufbringen des Polymers auf den Biosensor erfolgt bevorzugt durch Aufsprühen, Tauchcoaten oder Dispergieren einer verdünnten Lösung des Polymers, ist jedoch nicht auf diese Verfahren beschränkt. Als Lösungsmittel wird bevorzugt ein organisches Lösungsmittel, insbesondere ein organisches Lösungsmittel mit einem Siedepunkt von ≤ 100°C wie beispielsweise Ethanol, eingesetzt, welches eine Menge von etwa 0.1 Gew.% bis etwa 30 Gew.%, bevorzugt eine Menge von etwa 0.5 Gew.% bis etwa 15 Gew.%, an Polymer enthält. Polymere, welche für derartige Zwecke in Frage kommen, umfassen insbesondere Polymere, welche eine zwitterionische Struktur aufweisen und eine Mimikry von Zelloberflächen darstellen, wie beispielsweise 2-Methacryloyloxyethyl-Phosphorylcholin-con-Butyl-methacrylat (MPC-co-BMA). Die erhaltenen biokompatiblen Überzüge weisen üblicherweise eine Dicke von etwa 50 µm bis etwa 500 µm, bevorzugt von etwa 100 µm bis etwa 250 µm, auf.

Der zweite Abschnitt des elektrochemischen Sensors liegt in einem für das fluide Messmedium unzugänglichen Bereich und umfasst bevorzugt eine Messwerterfassungseinheit. In einer weiteren bevorzugten Ausführungsform umfasst der zweite Abschnitt weiterhin eine Spannungsquelle, wie beispielsweise eine Batterie oder einen Akkumulator, und ein Element, welches aus einer drahtlosen Datenübertragungseinheit und einem Display zur Messwertanzeige ausgewählt ist. Alternativ kann der zweite Abschnitt eine Schnittstelle für eine von dem elektrochemischen Sensor separate Messwerterfassungseinheit umfassen.

Bevorzugt ist der erfindungsgemäße elektrochemische Sensor zur Mehrfachmessung ausgebildet, d. h. der Sensor ermöglicht eine wiederholte Messung des zu bestimmenden Analyten. Dies ist insbesondere in Anwendungen erwünscht, in welche eine stetige, d. h. kontinuierliche oder diskontinuierliche Kontrolle des Vorhandenseins oder/und der Menge eines Analyten über einen längeren Zeitraum von z. B. 1 Tag oder länger, insbesondere 1 Woche oder länger, vorgenommen werden soll, wie beispielsweise bei Dialysepatienten. In einer bevorzugten Ausführungsform sieht die Erfindung demnach vor, dass der elektrochemische Sensor als Durchflusszelle ausgebildet ist, durch die ein den Analyten enthaltendes Fluid hindurchgeleitet wird. Alternativ kann der erfindungsgemäße elektrochemische Sensor allerdings auch als voll- oder teilimplantierbare Vorrichtung ausgebildet sein, welche beispielsweise in Fettgewebe oder in Blutgefäße implantiert werden kann.

Der erfindungsgemäße elektrochemische Sensor kann zur Bestimmung eines Analyten in einem fluiden Medium, welches aus einer beliebigen Quelle stammen kann, eingesetzt werden. In einer bevorzugten Ausführungsform dient der elektrochemische Sensor zur Bestimmung eines Analyten in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, wobei Vollblut, Plasma, Serum und extrazelluläre Gewebeflüssigkeit als besonders bevorzugt anzusehen sind. Die zur Durchführung der Analyse benötigte Probenmenge beträgt üblicherweise von etwa 0.01 µl bis etwa 100 µl, bevorzugt von etwa 0.1 µl bis etwa 2 µl.

Der qualtitativ oder/und quantitativ zu bestimmende Analyt kann eine beliebige biologische oder chemische Substanz sein, welche mittels einer Redoxreaktion nachgewiesen werden kann. Vorzugsweise wird der Analyt aus der Gruppe bestehend aus Äpfelsäure, Alkohol, Ammonium, Ascorbinsäure, Cholesterin, Cystein, Glucose, Glutathion, Glycerin, Harnstoff, 3-Hydroxybutyrat, Milchsäure, 5'-Nukleotidase, Peptiden, Pyruvat, Salicylat und Triglyceriden ausgewählt. In einer besonders bevorzugten Ausführungsform handelt es sich bei dem mittels des erfindungsgemäßen elektrochemischen Sensors zu bestimmenden Analyten um Glucose.

In einem weiteren Aspekt betrifft die vorliegende Erfindung ein Verfahren zur Herstellung eines erfindungsgemäßen elektrochemischen Sensors, umfassend die Schritte:
(a) Bereitstellen von Elektrokatalysatorpartikeln,
(b) Beschichten der Elektrokatalysatorpartikel mit einem Enzym, wobei das Enzym kovalent an die Elektrokatalysatorpartikel gebunden wird,
(c) Vermischen der in Schritt (b) erhaltenen, kovalent mit Enzym beschichteten Elektrokatalysatorpartikel mit einem leitfähigen Elektrodenmaterial und gegebenenfalls weiteren Substanzen,
(d) Verarbeiten des in Schritt (c) erhaltenen Gemisches zu einer Elektrode, und
(e) Kombinieren der in Schritt (d) erhaltenen Elektrode mit mindestens einer weiteren Elektrode.

Zum Zwecke der Herstellung der erfindungsgemäßen elektrochemischen Sensoren werden Partikel eines Elektrokatalysators, welcher wie vorstehend definiert ist, vorzugsweise zunächst mit einem Beschichtungsreagenz umgesetzt, wodurch die Elektrokatalysatorpartikel an ihrer Oberfläche funktionalisiert werden. Durch aufeinanderfolgende Umsetzung der funktionalisierten Elektrokatalysatorpartikel mit einem Vernetzungsreagenz und einem Enzym werden kovalent mit Enzym beschichtete Elektrokatalysatorpartikel erhalten, welche durch Vermischen mit weiteren Komponenten wie vorstehend definiert zu einer Elektrodenmatrix verarbeitet werden können.

Tatsächlich erweist sich das erfindungsgemäße Herstellungsverfahren als besonders vorteilhaft, da die Herstellung des mit Enzym beschichteten Elektrokatalysators getrennt von der Herstellung der Elektrode vorgenommen werden kann. Ferner steht mit dem kovalent mit Enzym beschichteten Elektrokatalysator ein definiertes Ausgangsmaterial für das Ansetzen der Elektrodenpaste zur Verfügung steht, welches vor Einbringung in die Elektrodenpaste gereinigt werden kann, so dass eine nachträgliche Reinigung der fertigen Elektrode entfällt.

In noch einem weiteren Aspekt betrifft die vorliegende Erfindung ein Verfahren zur Bestimmung eines Analyten in einem fluiden Medium, umfassend die Schritte:
(a) Inkontaktbringen des fluiden Mediums mit einem erfindungsgemäßen elektrochemischen Sensor, und
(b) Bestimmen des Vorhandenseins oder/und der Menge des Analyten in dem fluiden Medium durch Messung eines von dem elektrochemischen Sensor erzeugten Signals.

Zur Bestimmung des Analyten kann der erfindungsgemäße elektrochemische Sensor in einer beliebigen Art und Weise ausgestaltet sein, welche einen Kontakt zwischen dem elektrochemischen Sensor und dem fluiden Medium ermöglicht. So kann der Sensor beispielsweise als Durchflusszelle ausgebildet sein, durch welche das den Analyten enthaltende fluide Medium hindurchströmt. Andererseits kann der Sensor auch als Diffusionssensor ausgebildet sein, wobei der Kontakt zwischen Sensor und Medium durch Diffusion erfolgt. Gleichermaßen kann der elektrochemische Sensor als Vorrichtung ausgebildet sein, welche für eine vollständige oder teilweise Implantation in den Körper eines Patienten vorgesehen ist, wobei die Implantation entweder in ein Blutgefäß oder in Gewebe, insbesondere in subkutanes Fettgewebe, erfolgen kann.

Abhängig vom Vorhandensein oder/und der Menge des Analyten wird vom Sensor ein messbares Signal erzeugt. Bevorzugt handelt es sich bei diesem Signal um ein elektrisches Signal, wie beispielsweise elektrischen Strom, Spannung, Widerstand, etc., welches unter Verwendung geeigneter Mittel ausgewertet bzw. ausgelesen wird. Vorzugsweise ist der elektrochemische Sensor ein amperometrischer Sensor.

Die Erfindung soll durch die nachfolgenden Figuren und Beispiele näher erläutert werden.

### Abbildungen

Figur 1 zeigt den Umsatz an Glucose aufgetragen gegen die Glucosekonzentration [mM] bei Verwendung von Glucose-Oxidase als Enzym und Sauerstoff/Wasserstoffperoxid als Redoxmediator in Abhängigkeit von der Sauerstoffkonzentration. Kₘ^{app} und Vₘₐₓ^{app} stellen die nach einer Michaelis-Menten-Kinetik berechneten Enzymkinetikkonstanten für Glucose dar.

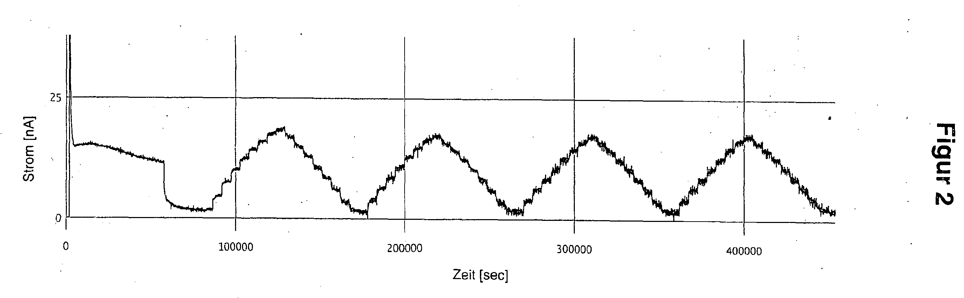

Figur 2 zeigt das Messsignal [nA] eines elektrochemischen Sensors gemäß vorliegender Erfindung aufgetragen gegen die Zeit [sec] im Rahmen einer 7-tägigen Messung der Glucosekonzentration einer Messlösung, in welcher die Glucosekonzentration periodisch zwischen 0 und 26 mM variiert wurde. Als Arbeitselektrode wurde eine Elektrode ohne Deckmembran verwendet, welche Glucose-Oxidase beschichteten Braunstein als Elektrokatalysator enthielt und gemäß Beispiel 6 der vorliegenden Anmeldung hergestellt wurde.

Figur 3 zeigt das Messsignal [nA] zweier elektrochemischer Sensoren mit immobilisiertem Enzym aufgetragen gegen die Glucosekonzentration einer Messlösung [mM]. Die als Dreiecke gekennzeichneten Messwerte zeigen die Funktionskurve eines elektrochemischen Sensors gemäß vorliegender Erfindung, in welchem Glucose-Oxidase kovalent an den Elektrokatalysator der Arbeitselektrode gebunden und keine Deckmembran verwendet wurde. Die als Quadrate gekennzeichneten Messwerte bilden die Funktionskurve eines Sensors identischer Dimension, in welchem das Enzym mittels einer aus Polyurethan bestehenden Deckmembran in der Arbeitselektrode immobilisiert und nicht über eine kovalente Bindung an den Elektrokatalysator gebunden wurde.

### Beispiele

### Beispiel 1: Darstellung von carboxyfunktionalisiertem Braunstein

Zur Darstellung von carboxyfunktionalisiertem Braunstein wurden 1.6 g Braunstein (Fa. Technipur) in 256 ml Toluol suspendiert, wurde die resultierende Suspension mit 84 g Geniosil^{®} GF 20 (Fa. Wacker) versetzt, und wurde das Reaktionsgemisch unter Stickstoffatmosphäre für 24 h bei 50°C und 520 U/min gerührt. Nach Abkühlen und Absitzen des Braunsteins wurde das Toluol abdekantiert und der Rückstand zweimal mit je 250 ml Toluol sowie anschließend einmal mit 250 ml Aceton gewaschen. Der auf diese Weise erhaltene funktionalisierte Braunstein wurde mit 250 ml Wasser versetzt und 24 h bei Raumtemperatur gerührt. Anschließend wurde das Wasser abzentrifugiert und der Rückstand unter Vakuum bei 50°C über CaCl₂ getrocknet, wobei ca. 1.5 mg an carboxyfunktionalisiertem Braunstein erhalten wurden.

### Beispiel 2: Kopplung von Glucose-Oxidase an carboxyfunktionalisierten Braunstein

100 mg des getrockneten, carboxyfunktionalisierten Braunsteins aus Beispiel 1 wurden mit 500 mg 1-Ethyl-3-(3-dimethylaminopropyl)carbodiimid (EDC), 400 mg N-Hydroxysuccinimid und 70 mg Glucose-Oxidase versetzt und in wässriger Lösung für 24 h bei Raumtemperatur gerührt. Nach Absitzen des Feststoffs wurde der Überstand entfernt und der Feststoff viermal mit Kaliumphosphatpuffer pH 7.4 gewaschen. Nach Trocknen des erhaltenen Feststoffes an Luft wurden ca. 85 mg an enzymbeschichtetem Elektrokatalysator erhalten, welcher eine Enzymaktivität von 0.06 U/mg aufwies.

### Beispiel 3: Darstellung von aminofunktionalisiertem Braunstein

Zu einer gut gerührten, auf 60°C temperierten Suspension von 200 mg Braunstein (Fa. Technipur) in 32 ml Toluol wurden 8 ml 3-Aminopropyltriethyoxysilan (Fa. Sigma) gegeben und weitere 16 h bei 60°C gerührt. Nach Absetzen des Feststoffes wurde der klare Überstand abdekantiert und der Feststoff 3 mal mit je 32 ml Toluol gewaschen. Der verbliebene Feststoff wurde an Luft getrocknet, wobei ca. 182 mg an aminofunktionalisiertem Braunstein erhalten wurden.

### Beispiel 4: Kopplung von Glucose-Oxidase an aminofunktionalisierten Braunstein unter Verwendung von Glutardialdehyd als Vernetzungsreagenz

Der getrocknete Feststoff aus Beispiel 3 wurde einmal mit 32 ml 50 mM Kaliumphosphatpuffer pH 7.4 gewaschen und anschließend in 16 ml 50 mM Kaliumphosphatpuffer pH 7.4 aufgenommen. Zu dieser Suspension wurden unter Rühren 16 ml einer 10%igen Glutardialdehyd-Lösung (Fa. Sigma) gegeben. Nach 1.5 h bei 25°C wurde die Reaktion abgebrochen. Der abgesetzte Feststoff wurde 3 mal mit jeweils 32 ml 50 mM Kaliumphosphatpuffer pH 7.4 gewaschen, in 16 ml des gleichen Puffers unter Rühren suspendiert und mit 16 ml einer Lösung von 0.5 mg/ml Glucose-Oxidase (Fa. Roche) in 50 mM Kaliumphosphatpuffer pH 7.4 versetzt. Diese Gemisch wurde 3 h bei 25°C gerührt. Nach Absitzen des Feststoffs wurde dieser 4 mal mit jeweils 16 ml 50 mM Kaliumphosphatpuffer pH 7.4 gewaschen. Nach Lyophilisation wurden ca. 200 mg an enzymbeschichtetem Elektrokatalysator erhalten, welcher eine Enzymaktivität von 0.12 U/mg aufwies.

### Beispiel 5: Kopplung von Glucose-Oxidase an aminofunktionalisierten Braunstein unter Verwendung von Disuccinimidylsuberat als Vernetzungsreagenz

20 mg des getrockneten, aminofunktionalisierten Braunsteins aus Beispiel 3 wurden mit 0.02 mg Disuccinimidylsuberat in 20 µl Dioxan sowie 0.008 mg Glucose-Oxidase in 2 ml 0.1 M Kaliumphosphatpuffer pH 8.5 versetzt und für 4 h bei Raumtemperatur gerührt. Nach Abzentrifugieren des Feststoffs wurde dieser 2 mal mit jeweils 5 ml 0.1 M Kaliumphosphatpuffer pH 8.5 gewaschen und anschließend in 5 ml 0.1 M Kaliumphosphatpuffer pH 8.5 aufgenommen. Nach Lyophilisation wurden ca. 18.8 mg an enzymbeschichtetem Elektrokatalysator erhalten, welcher eine Enzymaktivität von 0.1 U/mg aufwies.

### Beispiel 6: Herstellung von amperometrischen Sensoren

Zur Herstellung eines elektrochemischen Sensors mit drei Elektroden (Arbeitselektrode, Referenzelektrode und Gegenelektrode), welcher eine Bestimmung von Glucose in Blut oder subkutanem Fettgewebe gestattet, wurde im erstem Schritt eine Arbeitselektrode ohne Deckmembran hergestellt. Hierzu wurde mit Glucose-Oxidase funktionalisierter Braunstein gemäß Beispiel 5 mit Carbon-Polymerpaste PE 401 (Fa. Acheson) und Diethylenglykolmonobutylether vermischt, das erhaltene Gemisch mittels Dispensiertechnik auf die Goldfläche eines Sensorstreifens aus Polyester aufgebracht, und bei 25°C im Vakuum getrocknet. Die auf diese Weise erhaltene Arbeitselektrode wurde mit einer Silber/Silberchlorid-Elektrode als Referenzelektrode sowie einer Goldelektrode als Gegenelektrode kombiniert. Die Leiterbahnen waren isoliert.

### Beispiel 7: Bestimmung der Stabilität und Linearität des Messsignals amperometrischer Sensoren

Der gemäß Beispiel 6 erhaltene elektrochemische Sensor wurde in eine sich in einer Durchflusskammer befindliche Glucoselösung eingetaucht und über 7 Tage vermessen, wobei die Konzentration der Glucoselösung kontinuierlich zwischen 0 und 26 mM variiert wurde. Die Figuren 2 und 3 zeigen die Ergebnisse dieser Messung.

## Patentansprüche

1. Elektrochemischer Sensor zur Bestimmung eines Analyten in einem fluiden Medium umfassend mindestens eine Arbeitselektrode und mindestens eine Referenzelektrode, wobei mindestens die Arbeitselektrode Partikel eines Elektrokatalysators in einer Elektrodenmatrix umfasst,
**dadurch gekennzeichnet,**
**dass** ein zur Bestimmung des Analyten geeignetes Enzym kovalent an die Partikel des Elektrokatalysators gebunden ist.

2. Elektrochemischer Sensor nach Anspruch 1,
**dadurch gekennzeichnet,**
**dass** der Elektrokatalysator ein Metalloxid, insbesondere ein Metalloxid ausgewählt aus der Gruppe bestehend aus MnO₂, FeOOH, Fe₃O₄, Fe₂O₃, Cr₂O₃ und V₂O₅, ist.

3. Elektrochemischer Sensor nach Anspruch 1 oder 2,
**dadurch gekennzeichnet,**
**dass** der Elektrokatalysator MnO₂ ist.

4. Elektrochemischer Sensor nach einem der Ansprüche 1 bis 3,
**dadurch gekennzeichnet,**
**dass** 90% der Elektrokatalysatorpartikel einen Durchmesser von 0.1 µm bis 20 µm, insbesondere von 0.5 µm bis 5 µm, aufweisen.

5. Elektrochemischer Sensor nach einem der Ansprüche 1 bis 4,
**dadurch gekennzeichnet,**
**dass** die Elektrokatalysatorpartikel eine Enzymaktivität von etwa 0.01 U/mg bis etwa 10 U/mg, insbesondere von etwa 0.1 U/mg bis etwa 10 U/mg, aufweisen.

6. Elektrochemischer Sensor nach einem der Ansprüche 1 bis 5,
**dadurch gekennzeichnet,**
**dass** die Elektrokatalysatorpartikel eine funktionalisierte Oberfläche aufweisen, an die das Enzym gebunden ist.

7. Elektrochemischer Sensor nach Anspruch 6,
**dadurch gekennzeichnet,**
**dass** die Oberfläche der Elektrokatalysatorpartikel mit Aminogruppen oder/und Carboxylgruppen funktionalisiert ist.

8. Elektrochemischer Sensor nach Anspruch 6 oder 7,
**dadurch gekennzeichnet,**
**dass** die Oberfläche der Elektrokatalysatorpartikel mit einem Aminosilan, insbesondere mit 3-Aminopropyltriethoxysilan, funktionalisiert ist.

9. Elektrochemischer Sensor nach Anspruch 6 oder 7,
**dadurch gekennzeichnet,**
**dass** die Oberfläche der Elektrokatalysatorpartikel mit einem Carboxysilan, insbesondere mit 3-(Triethoxysilyl)propylbernsteinsäure-anhydrid, funktionalisiert ist.

10. Elektrochemischer Sensor nach einem der Ansprüche 6 bis 9,
**dadurch gekennzeichnet,**
**dass** das Enzym direkt an die funktionalisierte Oberfläche der Elektrokatalysatorpartikel gebunden ist.

11. Elektrochemischer Sensor nach einem der Ansprüche 6 bis 9,
**dadurch gekennzeichnet,**
**dass** das Enzym über ein Vernetzungsreagenz an die funktionalisierte Oberfläche der Elektrokatalysatorpartikel gebunden ist.

12. Elektrochemischer Sensor nach Anspruch 11,
**dadurch gekennzeichnet,**
**dass** das Vernetzungsreagenz ein Succinimid, insbesondere Disuccinimidylsuberat, ist.

13. Elektrochemischer Sensor nach einem der Ansprüche 1 bis 12,
**dadurch gekennzeichnet,**
**dass** die Elektrodenmatrix den Elektrokatalysator in einer Menge von etwa 1 Gew.% bis etwa 50 Gew.%, insbesondere in einer Menge von etwa 5 Gew.% bis etwa 20 Gew.%, enthält.

14. Elektrochemischer Sensor nach einem der Ansprüche 1 bis 13,
**dadurch gekennzeichnet,**
**dass** die Elektrodenmatrix porös ausgebildet ist.

15. Elektrochemischer Sensor nach einem der Ansprüche 1 bis 14,
**dadurch gekennzeichnet,**
**dass** die Elektrodenmatrix ferner ein leitfähiges Elektrodenmaterial umfasst.

16. Elektrochemischer Sensor nach einem der Ansprüche 1 bis 15,
**dadurch gekennzeichnet,**
**dass** das Enzym eine Oxidase ist.

17. Elektrochemischer Sensor nach einem der Ansprüche 1 bis 16,
**dadurch gekennzeichnet,**
**dass** das Enzym Glucose-Oxidase ist.

18. Elektrochemischer Sensor nach einem der Ansprüche 1 bis 17,
**dadurch gekennzeichnet,**
**dass** er ferner eine Gegenelektrode umfasst.

19. Elektrochemischer Sensor nach einem der Ansprüche 1 bis 18,
**dadurch gekennzeichnet,**
**dass** die Gegenelektrode mit einem leitfähigen Material beschichtet ist.

20. Elektrochemischer Sensor nach einem der Ansprüche 1 bis 19,
**dadurch gekennzeichnet,**
**dass** er mindestens zwei Abschnitte enthält, wobei der erste Abschnitt die Elektroden umfasst, mit einem biokompatiblen Überzug versehen ist und mit dem den Analyten enthaltenden fluiden Medium in Kontakt gebracht werden kann, und wobei der zweite Abschnitt in einem für das fluide Medium unzugänglichen Bereich liegt.

21. Elektrochemischer Sensor nach Anspruch 20,
**dadurch gekennzeichnet,**
**dass** der biokompatible Überzug durch Aufbringen einer vorgefertigten Membran auf den Sensor ausgebildet ist.

22. Elektrochemischer Sensor nach Anspruch 20,
**dadurch gekennzeichnet,**
**dass** der biokompatible Überzug durch Aufbringen einer Lösung eines Polymers auf den Sensor und anschließendes Trocknen ausgebildet ist.

23. Elektrochemischer Sensor nach Anspruch 22,
**dadurch gekennzeichnet,**
**dass** das Polymer 2-Methacryloyloxyethyl-Phosphorylcholin-co-n-Butylmethacrylat ist.

24. Elektrochemischer Sensor nach einem der Ansprüche 20 bis 23,
**dadurch gekennzeichnet,**
**dass** der biokompatible Überzug eine Dicke von etwa 50 µm bis etwa 500 µm, bevorzugt von etwa 100 µm bis etwa 250 µm, aufweist.

25. Elektrochemischer Sensor nach Anspruch 20,
**dadurch gekennzeichnet,**
**dass** der zweite Abschnitt eine Messwerterfassungseinheit umfasst.

26. Elektrochemischer Sensor nach Anspruch 25,
**dadurch gekennzeichnet,**
**dass** der zweite Abschnitt weiterhin eine Spannungsquelle und ein Element ausgewählt aus einer drahtlosen Datenübertragungseinheit und einem Display zur Messwertanzeige umfasst.

27. Elektrochemischer Sensor nach Anspruch 20,
**dadurch gekennzeichnet,**
**dass** der zweite Abschnitt eine Schnittstelle für eine von dem elektrochemischen Sensor separate Messwerterfassungseinheit umfasst.

28. Elektrochemischer Sensor nach einem der Ansprüche 1 bis 27,
**dadurch gekennzeichnet,**
**dass** er zur Mehrfachmessung ausgebildet ist.

29. Elektrochemischer Sensor nach einem der Ansprüche 1 bis 28,
**dadurch gekennzeichnet,**
**dass** er als voll- oder teilimplantierbare Vorrichtung ausgebildet ist.

30. Elektrochemischer Sensor nach einem der Ansprüche 1 bis 28,
**dadurch gekennzeichnet,**
**dass** er als Durchflusszelle ausgebildet ist.

31. Elektrochemischer Sensor nach einem der Ansprüche 1 bis 30 zur Bestimmung eines Analyten in einem Körperfluid, insbesondere in Vollblut, Plasma, Serum, oder extrazellulärer Gewebeflüssigkeit.

32. Elektrochemischer Sensor nach einem der Ansprüche 1 bis 31 zur Bestimmung eines Analyten ausgewählt aus der Gruppe bestehend aus Äpfelsäure, Alkohol, Ammonium, Ascorbinsäure, Cholesterin, Cystein, Glucose, Glutathion, Glycerin, Harnstoff, 3-Hydroxybutyrat, Milchsäure, 5'-Nukleotidase, Peptiden, Pyruvat, Salicylat und Triglyceriden, insbesondere von Glucose.

33. Verfahren zur Herstellung eines elektrochemischen Sensors nach einem der Ansprüche 1 bis 32, umfassend die Schritte:
(a) Bereitstellen von Elektrokatalysatorpartikeln,
(b) Beschichten der Elektrokatalysatorpartikel mit einem Enzym, wobei das Enzym kovalent an die Elektrokatalysatorpartikel gebunden wird,
(c) Vermischen der in Schritt (b) erhaltenen, kovalent mit Enzym beschichteten Elektrokatalysatorpartikel mit einem leitfähigen Elektrodenmaterial und gegebenenfalls weiteren Substanzen,
(d) Verarbeiten des in Schritt (c) erhaltenen Gemisches zu einer Elektrode, und
(e) Kombinieren der in Schritt (d) erhaltenen Elektrode mit mindestens einer weiteren Elektrode.

34. Verfahren nach Anspruch 33,
**dadurch gekennzeichnet,**
**dass** in Schritt (b) die Elektrokatalysatorpartikel zunächst mit einem Beschichtungsreagenz, anschließend mit einem Vernetzungsreagenz, und schließlich mit dem Enzym umgesetzt werden.

35. Verfahren zur Bestimmung eines Analyten in einem fluiden Medium, umfassend die Schritte:
(a) Inkontaktbringen des fluiden Mediums mit einem elektrochemischen Sensor nach einem der Ansprüche 1 bis 32, und
(b) Bestimmen des Vorhandenseins oder/und der Menge des Analyten in dem fluiden Medium durch Messung eines von dem elektrochemischen Sensor erzeugten Signals.
